# EUROPÄISCHE PATENTANMELDUNG

(11) **EP 3 178 827 A1**
(43) Veröffentlichungstag der Anmeldung: **14.06.2017**
(21) Anmeldenummer: 15198148.7
(22) Anmeldetag: 07.12.2015
(51) Int. Cl.: C07F 9/6574

(54) **HETEROZYKLISCHE SELENA-BIPHOSPHITE SOWIE VERFAHREN ZU DEREN HERSTELLUNG**

(71) Anmelder: Evonik Degussa GmbH, 45128 Essen (DE)
(72) Erfinder: DYBALLA, Katrin Marie, 45657 Recklinghausen (DE); FRANKE, Robert, 45772 Marl (DE); WEILBEER, Claudia, 06406 Bernburg (DE); SELENT, Detlef, 18059 Rostock (DE); BÖRNER, Armin, 18059 Rostock (DE)

(57) **Zusammenfassung**

Neue heterozyklische Selena-Bisphosphite, Verfahren zu deren Herstellung als auch deren Verwendung als Ligandenbaustein zur Herstellung von Liganden für eine Anwendung in Komplexen.

## Beschreibung

Neue heterozyklische Selena-Bisphosphite, Verfahren zu deren Herstellung als auch deren Verwendung als Ligandenbaustein zur Herstellung von Liganden für eine Anwendung in Komplexen.

T. K. Paine beschreibt eine Synthese von 2,2'-Selenobis(4,6-di-tert-butylphenol) unter Verwendung von Selendioxid. Die Herstellung von 2,2'-Selenobis(4,6-di-tert-butylphenol) erfolgt hier in einem sauren Medium unter Zusatz von konzentrierter Salzsäure. Das Produkt wird mit einer Ausbeute von 25 % erhalten (T. K. Paine et al., "Manganese complexes of mixed O, X, O-donor ligands (X = S or Se): synthesis, characterization and catalytic reactivity", Dalton Trans., 2003, 15, 3136-3144). Hierbei ist besonders nachteilig, dass die Ausbeuten sehr gering und somit verbesserungswürdig sind.

Eine weitere mehrstufige Syntheseroute unter Verwendung von Grignard-Reagenz offenbart H. M. Lin et al., "A novel and efficient synthesis of selenides", ARKIVOC, 2012, viii, 146-156. Es wird eine Syntheseroute für Selenobiarylether offenbart in der zunächst Brom an das entsprechende Phenol addiert werden muss, um das Produkt dann mit Magnesium zu einem Grignard-Reagenz umzusetzen. Das Grignard-Reagenz kann dann mit dem zugesetzten Selen reagieren, bevor die eigentliche Kupplung zum Biarylether erfolgt:

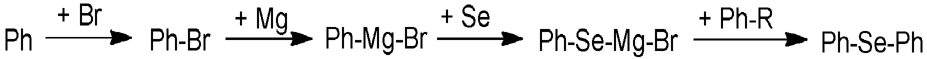

Das Produkt konnte in einer guten Ausbeute erzielt werden, jedoch ist diese Syntheseroute sehr aufwendig, was es für einen grosstechnischen Einsatz unattraktiv macht. Hierbei sind eine Vielzahl von Syntheseschritten notwendig, die zum Teil nicht unkritisch in ihrer Durchführung sind, insbesondere wenn man an eine Hochskalierung denkt und Massstäbe verwendet, die in der Industrie üblich sind. Weiterhin fallen bei dieser Syntheseroute große Mengen an Abfallprodukten und Lösungsmittel an, die aufwändig entsorgt werden müssen, unter anderem auf Grund des Einsatzes von Brom.

Eine grosstechnisch wirtschaftliche Syntheseroute zur Herstellung von Selenodiphenolen beschreibt die EP 15168645.8 bzw. US 14/720,063.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxidierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Rhodium-Monophosphit-Komplexe in katalytisch aktiven Zusammensetzungen sind geeignet für die Hydroformylierung von verzweigten Olefinen mit innenständigen Doppelbindungen. Seit den 1970er Jahren ist die Verwendung von so genannten "bulky phosphites" in der Hydroformylierung beschrieben (siehe u.a. van Leeuwen et al., Journal of Catalysis, 2013, 298, 198-205). Diese zeichnen sich durch eine gute Aktivität aus, jedoch ist die n/i-Selektivität für endständig oxidierte Verbindungen gering und verbesserungswürdig.

In diesen Hydroformylierungen werden in der Regel Mono- und Bisphosphite eingesetzt, die oftmals aus Biphenol-Bausteinen aufgebaut sind. Die Entwicklung neuer Liganden ist häufig durch die zur Verfügung stehenden Biphenol-, also Ligandenbausteine limitiert. So stellen 2,2'-Selenobiarylether sowie Diphenylselenoxide und Diphenylselenide eine hochinteressante Verbindungsklasse dar. Die 2,2'-Selenobiarylether werden derzeit nur in bestimmten Komplexen, vor allem manganhaltigen, verwendet, sie besitzen aber ein großes Potential für weitere Anwendungen.

Aufgabe der Erfindung war es, eine weitere gänzlich neue Substanzklasse an Liganden und Ligandenbausteinen herzustellen, um das Feld der verfügbaren Liganden für die jeweiligen spezifischen Komplexe in der Katalyse zu erweitern. Des Weiteren bestand die Aufgabe Liganden für Rhodium-Hydroformylierungskatalysatoren herzustellen. Daher bestand auch die Aufgabe neue Zwischenprodukte als Ligandenbausteine zur Herstellung von Liganden bereitzustellen. Gelöst werden die Aufgaben mit den heterozyklischen Selena-Phosphiten nach Anspruch 1, dem Verfahren nach Anspruch 7 sowie der Verwendung nach Anspruch 15. Besondere Ausführungsformen sind in den Unteransprüchen sowie detailliert in der Beschreibung offenbart. Gelöst werden die Aufgaben vorzugsweise mit Selena-Phosphiten der Strukturen **I** und **Ia**, insbesondere mit R¹ und/oder R^{1*} ausgewählt aus Struktur **II, IV, V, VI,** und **VII.** Dabei sind die Wasserstoff, Alkyl und/oder -O-(C₁-C₁₂)-Alkyl substituierten Verbindungen von R¹ und R^{1*} der genannten Strukturen besonders bevorzugte Verbindungen.

Gegenstand der Erfindung sind Verbindungen mindestens eines heterozyklischen Selena-Phosphites, welches eine allgemeine Struktur **I** aufweist 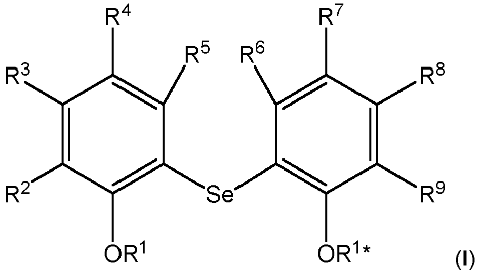 wobei R², R³, R⁴, R⁵, R⁶, R⁷, R⁸ und R⁹ jeweils unabhängig voneinander ausgewählt sind aus: -H,-(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sein können, insbesondere umfassend die Alkyl- und/oder Arylgruppen in -(C₁-C₁₂)-Alkyl, -O-(C₁-C₁₂)-Alkyl, -(C₆-C₂₀)-Aryl, -O-(C₆-C₂₀)-Aryl, -OC=O-(C₁-C₁₂)-Alkyl, wobei die jeweilige substituierte -(C₁-C₁₂)-Alkylgruppe und substituierte -(C₆-C₂₀)-Arylgruppe, mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei die Gruppen -R¹ und -R^{1*} unabhängig ausgewählt sind aus -H und mindestens einer organofunktionellen Phosphit-Gruppe synonym zu Phosphorigsäureester, wobei mindestens eine Gruppe von -R¹ und -R^{1*} einer organofunktionellen Phosphit-Gruppe entspricht. Gleichfalls Gegenstand der Erfindung sind Zusammensetzungen umfassend mindestens ein Selena-Phosphit der Struktur **I** sowie Gemische von Selena-Phosphiten der Struktur **I**, wie beispielsweise der nachfolgend dargestellten Strukturen **I**, **I**** und/oder **I**** 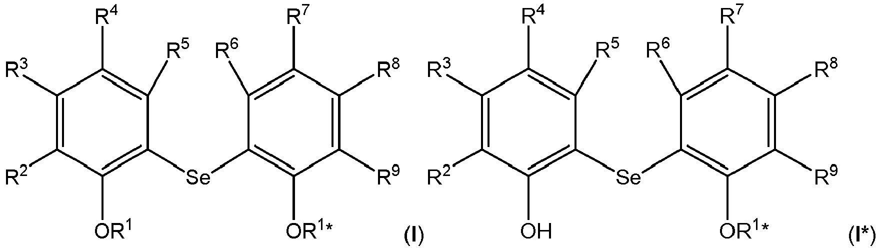 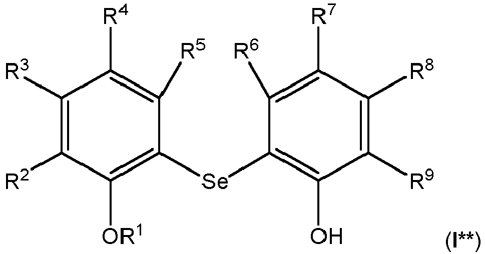 mit R², R³, R⁴, R⁵, R⁶, R⁷, R⁸ und R⁹, -R¹ und -R^{1*} entsprechend vorstehend oder nachstehend definierter Bedeutung. Die Herstellung der Verbindungen der Strukturen I* und I** kann insbesondere interessant sein, wenn die Strukturen als Ligandenbaustein verwendet werden sollen.

Die Alkylgruppen können grundsätzlich linear, verzweigt oder cyclisch sein. Nach einer bevorzugten Alternative können die Alkyl- und Arylgruppen jeweils unabhängig unsubstituiert sein.

Nach einer bevorzugten Alternative sind in der allgemeinen Struktur I die Gruppen -R¹ und -R^{1*} unabhängig ausgewählt aus den Strukturen **II**, **III**, **IV**, **V**, **VI** und **VII**.

Entsprechend einer besonders bevorzugten Alternative sind in der allgemeinen Struktur I die Gruppen -R¹ und -R^{1*} gleich und ausgewählt aus den Strukturen **II**, **III**, **IV**, V, **VI** und **VII**. Ferner sind Gegenstand der Erfindung Gemische der Verbindung der Struktur **I** in denen -R¹ und -R^{1*} ausgewählt sind aus -H und einer Struktur von **II**, **III**, **IV**, **V**, **VI** und **VII**.

Nach einer besonders bevorzugten Alternative kann in dem heterozyklischen Selena-Phosphit der allgemeinen Struktur **I**
a) mindestens eine Gruppe von -R¹ und/oder -R^{1*} einer organofunktionellen Phosphit-Gruppe und unabhängig ausgewählt aus den Strukturen **II**, **III**, **IV**, **V**, **VI** und **VII** entsprechen, 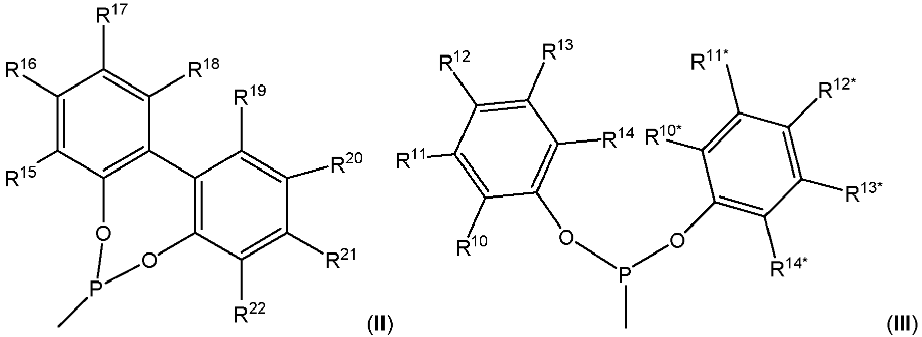 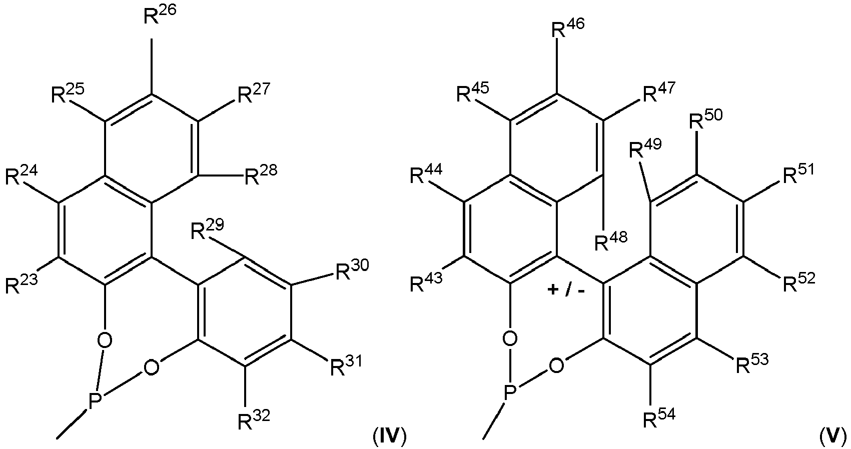 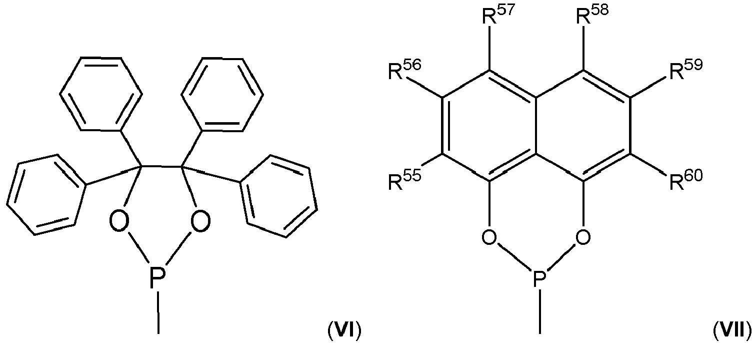 wobei die Reste
   R¹⁵ R¹⁶, R¹⁷, R¹⁸, R¹⁹, R²⁰, R²¹, R²²-in Struktur **II**,
   R¹⁰, R¹¹, R¹², R¹³, R¹⁴, R^{10*}, R^{11*}, R^{12*} R^{13*}, R^{14*} in der Struktur **III**,
   R²³, R²⁴, R²⁵, R²⁶, R²⁷, R²⁸, R²⁹, R³⁰, R³¹ und R³² in Struktur **IV**,
   R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur **V**,
   R⁵⁵, R⁵⁶, R⁵⁷, R⁵⁸, R⁵⁹ und R⁶⁰ in Struktur **VII,** in der jeweiligen Struktur jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, und/oder
b) -R¹ und -R^{1*} gleich sind und einer organofunktionellen Phosphit-Gruppe entsprechen können und ausgewählt sein können aus den Strukturen **II**, **III**, **IV**, V, **VI** und **VII,** oder
c) das Selena-Phosphit der Struktur **I** als Gemisch von a) und b) vorliegt.

Bevorzugt sind -R¹ und -R^{1*} gleich und entsprechen einer organofunktionellen Phosphit-Gruppe und sind ausgewählt aus den Strukturen **II**, **IV**, V, **VI** und **VII**.

Gleichfalls Gegenstand der Erfindung sind heterozyklische Selena-Phosphite der allgemeinen Struktur I ausgewählt aus mindestens einer Verbindung der Struktur **Ia** 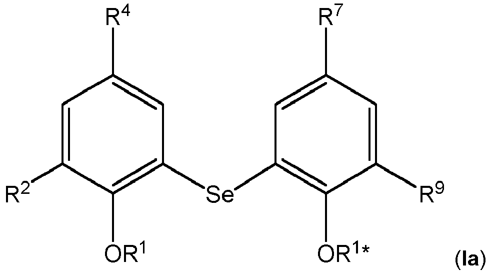 wobei R², R⁴, R⁷ und R⁹ jeweils unabhängig voneinander ausgewählt sein können aus: -(C₁-C₁₂)-Alkyl, -O-(C₁-C₁₂)-Alkyl, -(C₆-C₂₀)-Aryl, -O-(C₆-C₂₀)-Aryl, -Halogen, wobei -R¹ und -R^{1*} in Struktur **Ia** unabhängig ausgewählt sind aus -H und mindestens einer organofunktionellen Phosphit-Gruppe, wobei mindestens -R¹ oder -R^{1*} einer organofunktionellen Phosphit-Gruppe entspricht. Bevorzugt sind -R¹ und -R^{1*} gleich und entsprechen einer organofunktionellen Phosphit-Gruppe. In einer Alternative ist -R¹ oder -R^{1*} gleich -H und das verbleibende -R¹ oder -R^{1*} entspricht einer organofunktionellen Phosphit-Gruppe. Die zuletzt genannten Verbindungen können bspw. auch als Ligandenbaustein verwendet werden. Diese Verbindungen sind als Ligandenbaustein geeignet, da sie eine OH-Gruppe enthalten, die durch den Fachmann weiter umgesetzt werden kann, wodurch Liganden erhalten werden können.

Nach einer weiteren Ausführungsform ist Gegenstand der Erfindung mindestens ein heterozyklisches Selena-Phosphit der allgemeinen Struktur **I** oder **Ia** mit -R¹ und -R^{1*} ausgewählt aus den Strukturen **II**, **III**, **IV**, **V, VI** und **VII**, wie vorstehend dargestellt, wobei die Reste R¹⁵, R¹⁶, R¹⁷, R¹⁸, R¹⁹, R²⁰, R²¹, R²²-in Struktur **II**, R¹⁰, R¹¹, R¹², R¹³, R¹⁴, R^{10*}, R^{11*}, R^{12*}, R^{13*}, R^{14*} in der Struktur **III**, R²³, R²⁴, R²⁵, R²⁶, R²⁷, R²⁸, R²⁹, R³⁰, R³¹ und R³² in Struktur **IV,** R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur **V,** R⁵⁵, R⁵⁶, R⁵⁷, R⁵⁸, R⁵⁹ und R⁶⁰ in Struktur **VII,** jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, insbesondere sind die Reste ausgewählt aus -H, - (C₁-C₁₂)-Alkyl, -O-(C₁-C₁₂)-Alkyl, oder aus den Alternativen a) -H und -(C₁-C₁₂)-Alkyl, b) -H und -O-(C₁-C₁₂)-Alkyl, oder c) -H.

Gleichfalls Gegenstand der Erfindung sind heterozyklische Selena-Phosphite der allgemeinen Struktur I ausgewählt aus mindestens einer Verbindung der Struktur **Ia** mit -R¹ und -R^{1*} ausgewählt aus der Struktur **II** 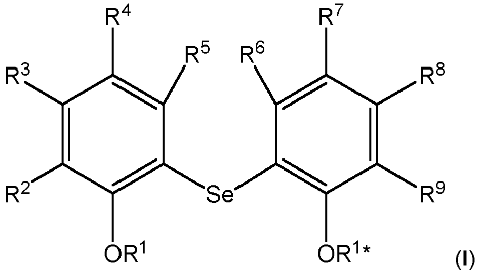 wobei R², R³, R⁴, R⁵, R⁶, R⁷, R⁸ und R⁹ jeweils unabhängig voneinander ausgewählt sind aus: -H,-(C₁-C₁₂)-Alkyl, -O-(C₁-C₁₂)-Alkyl, oder 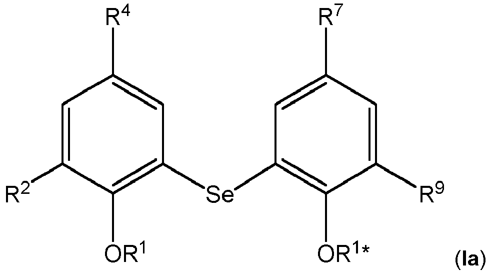 wobei R², R⁴, R⁷ und R⁹ jeweils unabhängig voneinander ausgewählt sind aus: -(C₁-C₁₂)-Alkyl, -O-(C₁-C₁₂)-Alkyl, und 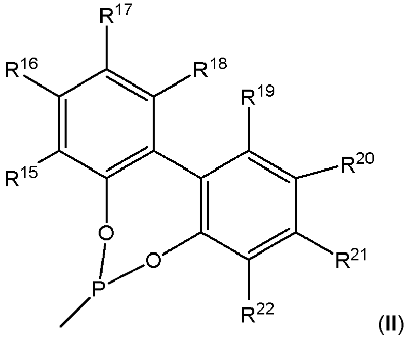 mit R¹⁵, R¹⁶, R¹⁷, R¹⁸, R¹⁹, R²⁰, R²¹, R²² in Struktur **II**, jeweils unabhängig voneinander ausgewählt aus: -H, -(C₁-C₁₂)-Alkyl, -O-(C₁-C₁₂)-Alkyl. Insbesondere mit R¹⁵, R¹⁶, R¹⁷, R¹⁸, R¹⁹, R²⁰, R²¹, R²² in Struktur **II** ausgewählt aus -H, -(C₁-C₁₂)-Alkyl, bevorzugt sind alle Reste R¹⁵ bis R²² -H.

Dabei kann es bevorzugt sein, wenn -R¹ und -R^{1*} in Struktur **I** oder **Ia** unabhängig ausgewählt sind aus -H und mindestens einer organofunktionellen Phosphit-Gruppe, wobei mindestens eine Gruppe von -R¹ und -R^{1*} einer organofunktionellen Phosphit-Gruppe entspricht.

Weitere besonders bevorzugte Alternativen umfassen heterozyklische Selena-Phosphite mit -R¹ und -R^{1*} ausgewählt aus den Alternativen
a) -R¹ und -R^{1*} sind in Struktur **I** oder **Ia** gleich und ausgewählt aus **II**, **III**, **IV**, **V**, **VI** und **VII**, und/oder
b) in Struktur **I** oder **Ia** ist -R¹ gleich -H und -R^{1*} ist ausgewählt aus **II**, **III**, **IV**, **V**, **VI** und **VII**, und/oder
c) in Struktur **I** oder **Ia** ist -R^{1*} gleich -H und -R¹ ist ausgewählt aus **II**, **III**, **IV**, **V**, **VI** und **VII**, und/oder
d) die Verbindung der Alternative a) liegt im Gemisch mit b) und/oder c) vor.

In Struktur **Ia** können R², R⁴, R⁷ und R⁹ jeweils unabhängig voneinander ausgewählt sein aus: - Methyl, -Ethyl,- *tert*-Butyl, -iso-Pentyl, -Methoxy, -Benzyl-; -Halogen.

In der Struktur **II** können R¹⁵, R¹⁶, R¹⁷, R¹⁸, R¹⁹, R²⁰, R²¹, R²² jeweils unabhängig ausgewählt sein aus: -H, -Methyl, -Ethyl, -*tert*-Butyl, -iso-Pentyl, -Methoxy, -Benzyl; -Halogen.

In der Struktur **III** können R¹⁰, R¹¹, R¹², R¹³, R¹⁴, R^{10*}, R^{11*}, R^{12*}, R^{13*}, R^{14*} jeweils unabhängig ausgewählt sein aus: -H, -Methyl, -Ethyl, -*tert*-Butyl, -iso-Pentyl, -Methoxy, -Benzyl; -Halogen.

In der Struktur **VII** können R⁵⁵, R⁵⁶, R⁵⁷, R⁵⁸, R⁵⁹ und R⁶⁰ jeweils unabhängig ausgewählt sein aus: - H, -Methyl, -Ethyl,- *tert*-Butyl, -iso-Pentyl, -Methoxy, -Benzyl-; -Halogen.

Gleichfalls Gegenstand der Erfindung ist ein Verfahren zur Herstellung mindestens eines heterozyklischen Selena-Phosphites der allgemeinen Struktur **I** sowie ein heterozyklisches Selena-Phosphit der Struktur **I** erhältlich nach dem Verfahren oder eine Zusammensetzung umfassend Gemische von heterozyklischen Selena-Phosphiten der Struktur **I** erhältlich nach dem Verfahren, 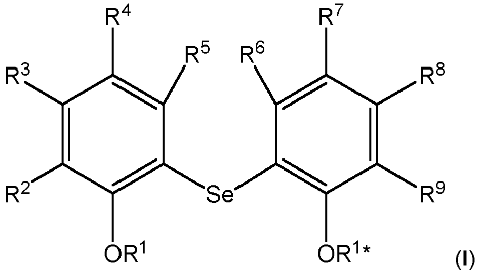 wobei R², R³, R⁴, R⁵, R⁶, R⁷, R⁸ und R⁹ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte - (C₆-C₂₀)-Arylgruppe, mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei die Gruppen -R¹ und - R^{1*} in Struktur **I** unabhängig ausgewählt sein können aus -H und mindestens einer organofunktionellen Phosphit-Gruppe, wobei mindestens eine Gruppe von -R¹ und -R^{1*} einer organofunktionellen Phosphit-Gruppe entsprechen kann,
umfassend mindestens den Verfahrensschritt
(i) Umsetzen eines Selenodiaryls der allgemeinen Struktur IX 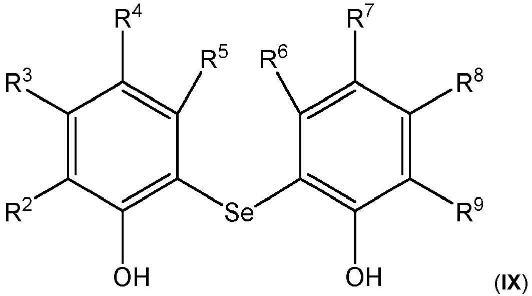 wobei R², R³, R⁴, R⁵, R⁶, R⁷, R⁸ und R⁹ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe, mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
(ii) mit mindestens einer Halogenphosphit-Verbindung R¹Hal der Formel **Xa** und/oder R^{1*}Hal der Formel **Xb,** wobei Hal jeweils unabhängig ausgewählt ist aus Fluor, Chlor, Brom, Jod, bevorzugt ist Chlor, wobei -R¹ und -R^{1*} in Formel **Xa** und/oder **Xb** jeweils unabhängig einer organofunktionellen Phosphit-Gruppe entsprechen, bevorzugt sind -R¹ und -R^{1*} gleich, sodass vorzugsweise R¹Hal der Formel **Xa** gleich R^{1*}Hal der Formel **Xb** ist,
(ii) und erhalten mindestens eines heterozyklischen Selena-Phosphits der allgemeinen Struktur **I** oder optional eines Gemisches von Selena-Phosphiten der Struktur **I**.

Die Hydroxyl-funktionellen heterozyklischen Selena-Phosphite mit R¹ oder R^{1*} gleich -H in den Strukturen, **I***, **I** I**, **Ia** können vorzugsweise durch eine unvollständige Umsetzung des Selenadiaryls der Struktur **IX** erhalten werden.

Nach einer bevorzugten Alternative kann in dem Verfahren mindestens eine Halogenphosphit-Verbindung R¹Hal der Formel **Xa** und/oder R^{1*}Hal der Formel **Xb** eingesetzt werden, in der Hal gleich Chlor ist und
a) -R¹ oder -R^{1*} jeweils unabhängig ausgewählt sein können aus den Strukturen **II**, **III**, **IV**, **V**, **VI** und **VII** 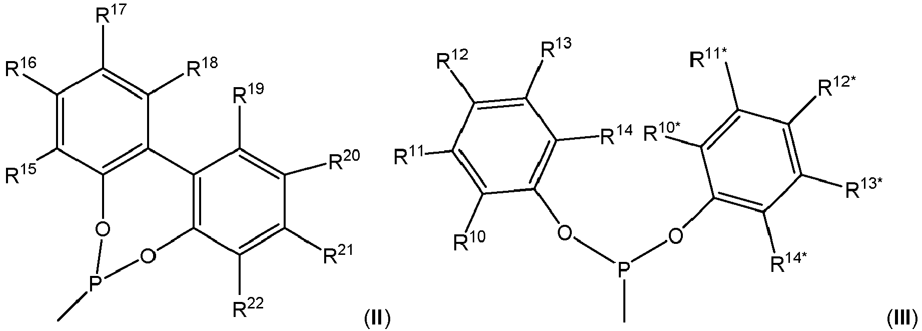 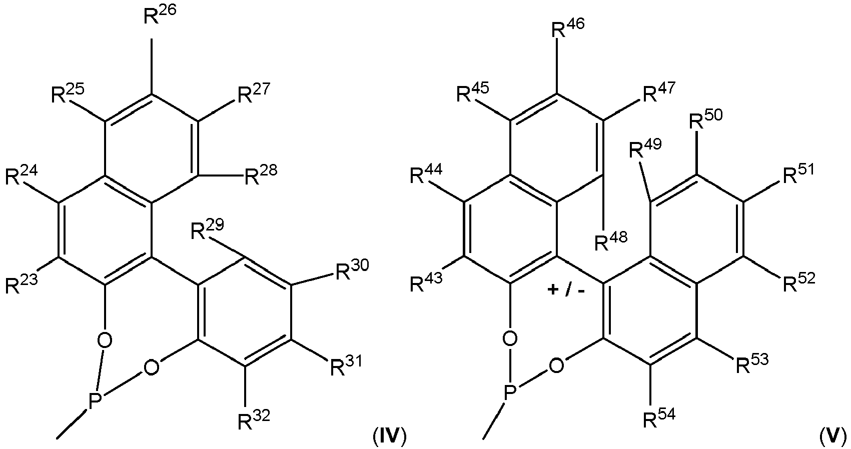 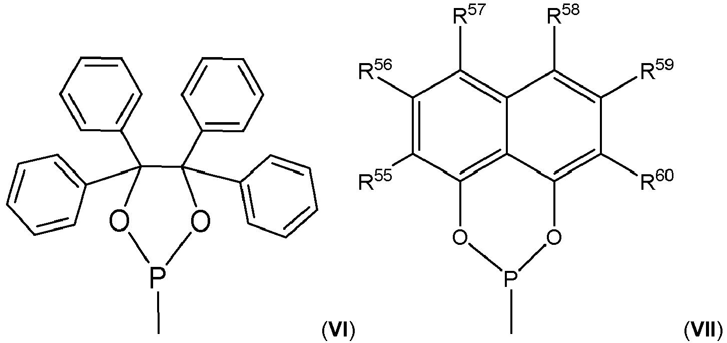 wobei die Reste
   R¹⁵ R¹⁶, R¹⁷, R¹⁸, R¹⁹, R²⁰, R²¹, R²² -in Struktur **II**, R¹⁰, R¹¹, R¹², R¹³, R¹⁴, R^{10*}, R^{11*}, R^{12*}, R^{13*}, R^{14*} in der Struktur **III,** R²³, R²⁴, R²⁵, R²⁶, R²⁷, R²⁸, R²⁹, R³⁰, R³¹ und R³² in Struktur **IV,** R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur V, R⁵⁵, R⁵⁶, R⁵⁷, R⁵⁸, R⁵⁹ und R⁶⁰ in Struktur **VII,** in der jeweiligen Struktur jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, und/oder b) -R¹ und -R^{1*} gleich sein können und ausgewählt sein können aus den Strukturen **II**, **III**, **IV**, **V**, **VI**, **VI** und **VII**.

Ebenso Gegenstand der Erfindung ist ein Verfahren in dem eine Halogenphosphit-Verbindung eingesetzt werden kann, mit der Massgabe, dass R¹Hal der Formel **Xa** und R^{1*}Hal der Formel **Xb** gleich sind, und Hal Chlor ist und
-R¹ und -R^{1*} ausgewählt sind aus den Strukturen **II, III, IV, V, VI** und **VII**, und wobei die Reste R¹⁵, R¹⁶, R¹⁷, R¹⁸, R¹⁹, R²⁰, R²¹, R²² in Struktur **II**, R¹⁰, R¹¹, R¹², R¹³, R¹⁴, R^{10*}, R^{11*}, R^{12*}, R^{13*}, R^{14*} in der Struktur **III,** R²³, R²⁴, R²⁵, R²⁶, R²⁷, R²⁸, R²⁹, R³⁰, R³¹ und R³² in Struktur **IV,** R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur **V,** R⁵⁵, R⁵⁶, R⁵⁷, R⁵⁸, R⁵⁹ und R⁶⁰ in Struktur **VII,** jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen.

Nach einer weiteren Alternative kann in dem Verfahren bevorzugt ein Selenodiaryl der allgemeinen Struktur **IXa** eingesetzt werden, 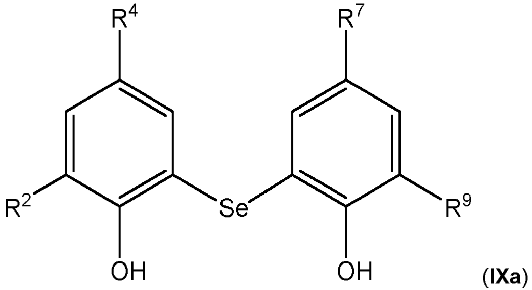 wobei R², R⁴, R⁷ und R⁹ jeweils unabhängig voneinander ausgewählt sein können aus: -(C₁-C₁₂)-Alkyl, -O-(C₁-C₁₂)-Alkyl, -(C₆-C₂₀)-Aryl, -O-(C₆-C₂₀)-Aryl, -Halogen.

Nach einer bevorzugten Verfahrensvariante erfolgt (i) die Umsetzung in Gegenwart einer Base, insbesondere eines Amins oder einer Pyridinbase, insbesondere eines Alkylamins, wie Triethylamin oder Dimethylaminobutan, insbesondere Triethylamin .

Zur Umsetzung werden das Selenodiaryl der allgemeinen Struktur **IX** oder **IXa** vorzugsweise mit R¹Hal der Formel **Xa** und R^{1*}Hal der Formel **Xb,** insbesondere sind **Xa** und **Xb** gleich, im molaren Verhältnis von 10 : 1 bis 1 : 10 umgesetzt, vorzugsweise erfolgt die Umsetzung im molaren Verhältnis von 4 : 1 bis 1 : 4, bevorzugt von 2,5 :1 bis 1 : 2,5. Wobei es weiter bevorzugt ist, wenn Hal gleich Chlor oder Brom ist.

Ferner wird die (i) Umsetzung vorzugsweise im Temperaturbereich von -45 bis 80 °C durchgeführt, besonders von -15 bis 30°C, insbesondere von -5 bis 25°C.

Zudem wird die (i) Umsetzung vorzugsweise in einem aprotischen Lösemittel durchgeführt, insbesondere kann das Lösemittel ausgewählt sein aus organischen aromatischen, halogenierten Lösemitteln oder Kohlenwasserstoffen

Ebenso ist Gegenstand der Erfindung die Verwendung eines heterozyklischen Selena-Phosphits als Ligand in einem Komplex umfassend mindestens ein Metallatom, insbesondere wenn -R¹ und -R^{1*} einer organofunktionellen Phosphit-Gruppe entsprechen, besonders bevorzugt kann die Verbindung der Struktur **I** oder **Ia** zur Katalyse einer Hydroformylierungsreaktion verwendet werden. Alternativ kann ein heterozyklisches Selena-Phosphit, indem -R^{1*} oder -R¹ gleich -H sind und die verbleibende Gruppe einer organofunktionellen Phosphit-Gruppe entspricht, als Zwischenprodukt zur Herstellung von Liganden verwendet werden.

Gleichfalls Gegenstand der Erfindung ist ein Komplex umfassend
- mindestens eine Verbindung der allgemeinen Struktur (**I**) oder (**Ia**) und
- mindestens ein Metallatom ausgewählt aus Rh, Ru, Co, Ir.

Ferner ist Gegenstand der Erfindung ein Verfahren umfassend die Verfahrensschritte
(i) Vorlegen mindestens eines Olefins,
(ii) Zugabe eines Komplexes umfassend
   - mindestens eine Verbindung der allgemeinen Struktur (**I**) oder (**Ia**) und
   - mindestens ein Metallatom ausgewählt aus Rh, Ru, Co, Ir, oder einer Verbindung der allgemeinen Struktur (**I**) oder (**Ia**) und einer Substanz, welche ein Metallatom ausgewählt aus Rh, Ru, Co, Ir aufweist, bevorzugt Rh, Ir, Ru, besonders bevorzugt Rh,
(iii) Zuführen von H₂ und CO,
(iv) Erwärmen des Reaktionsgemisches,
wobei das Olefin zu einem Aldehyd umgesetzt wird.

Die Verfahrensschritte (i), (ii), (ii) und (iv) können in jeder beliebigen Reihenfolge durchgeführt werden.

Der Begriff Phenol, Aryl, Phosphit wird in dieser Anmeldung als Gattungsbegriff verwendet und umfasst somit auch substituierte Strukturen der genannten Verbindungen.

Die vorgenannten Definitionen für substituierte -(C₁-C₁₂)-Alkylgruppen und substituierte -(C₆-C₂₀)-Arylgruppen gelten für alle Gruppen in denen diese Alkyl- oder Arylgruppen enthalten sind, also insbesondere auch für die folgenden Gruppen: -O-(C₁-C₁₂)-Alkyl, -(C₆-C₂₀)-Aryl, -O-(C₆-C₂₀)-Aryl, -OC=O-(C₁-C₁₂)-Alkyl.

Ein oder mehrere Substituenten in den vorgenannten Strukturen der Selena-Phosphite und Selenodiarylen umfassen vorzugsweise 1 bis 10 Substituenten, insbesondere 1 bis 3.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl-, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Halogen als Substituent an Alkyl oder Aryl umfasst Fluor, Chlor, Brom und Jod, wobei Chlor und Fluor besonders bevorzugt sind.

Alle Erläuterungen zum Ausdruck -(C₁-C₁₂)-Alkyl in den vorgenannten erfindungsgemäßen Strukturen der Selena-Phosphite und Selenodiarylen gelten auch für die Alkylgruppen in -O-(C₁-C₁₂)-Alkyl, also in -(C₁-C₁₂)-Alkoxy.

Bevorzugt sind unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen in den vorgenannten Strukturen der Selena-Phosphite und Selenodiarylen können in Abhängigkeit von ihrer Kettenlänge, einen oder mehrere Substituenten aufweisen. Die Substituenten sind vorzugsweise unabhängig voneinander ausgewählt aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl. Diese Definition gilt für alle substituierten Alkyl- oder Alkyoxygruppen der vorliegenden Erfindung.

Alle Erläuterungen zum Ausdruck -(C₆-C₂₀)-Aryl in den vorgenannten erfindungsgemäßen Strukturen der Selena-Phosphite und Selenodiarylen gelten auch für die Arylgruppen in -O-(C₆-C₂₀)-Aryl.

Bevorzugt sind unsubstituierte -O-(C₆-C₂₀)-Gruppen.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltigen Gruppen sind vorzugsweise ausgewählt aus -O-, -S-, -N-, -N(=O)-, -C(=O)- oder -S(=O)-. Beispiele für -(C₃-C₁₂)-Heterocycloalkylgruppen sind Tetrahydrothiophenyl, Tetrahydrofuryl, Tetrahydropyranyl und Dioxanyl.

Nachfolgend wird die Erfindung näher an Beispielen erläutert, ohne die Erfindung auf die Ausführungsbeispiele zu beschränken.

### Allgemeine Methoden

### Lösungsmittel und Reagenzien

Alle Reaktionen mit feuchtigkeits- und/oder sauerstoffempfindlichen Substanzen wurden in ausgeheizten Apparaturen unter Argonatmosphäre durchgeführt. Lösungsmittel zur Extraktion und Säulenchromatographie wurden in folgenden Reinheiten verwendet: Dichlormethan (99.9%, Fa. *Walter,* Art.-Nr. BIE 073107033) Ethylacetat (99.5%, Fa. *Walter,* Art.-Nr. BIE 003917025) und *n-*Hexan (95%, Fa. *Walter (Baker),* Art.-Nr. 8669),
n-Heptan (95%, Fa. *Walter (Baker),* Art.-Nr. 8662). Andere Lösungsmittel für Extraktion und Säulenchromatographie waren von technischer Qualität und wurden, sofern nicht anders vermerkt, ohne weitere Reinigung eingesetzt. Trockene Lösungsmittel (abs.) wurden mit einem Pure Solv MD-7 System gereinigt und unter Argonatmosphäre aufbewahrt. Benzylbromid wurde vor dem Gebrauch frisch destilliert (17 mbar/82°C). Deuterierte Lösungsmittel wurden von den angegebenen Trockenmitteln destilliert: Dichlormethan-d₂ (Phosphorpentoxid), Toluol-d₈ (1. KOH; 2. Natrium). Für die Synthesen wurden Chemikalien der Firmen *Sigma Aldrich, Alfa Aesar, Acros Organics, Avantor Performance Materials B.V., Merck KGaA* und *ABCR GmbH* & *Co. KG* verwendet. Diese wurden, sofern nicht anders vermerkt, ohne weitere Reinigung eingesetzt.

### Analytik

IR-Spektroskopie: Die IR-Spektren wurden mit dem FT-IR-Spektrometer *Nicolet 6700* der Firma *Thermo Electron* aufgenommen. Die Substanzen wurden mittels ATR-Verfahren vermessen.

¹H-NMR-Spektroskopie: Die ¹H-NMR-Spektren wurden mit dem Modell *AV 300* (300 MHz) sowie mit dem Modell *Fourier 300* (300 MHz) der Firma *Bruker* aufgenommen. Die chemischen Verschiebungen sind in Einheiten der δ-Skala angegeben. Die Restprotonensignale der Lösungsmittel (Dichlormethan-d₂: δ = 5.32 ppm, Toluol-d₈: δ = 7.09; 7.00; 6.98; 2.09 ppm) dienten dabei als Standard.

¹³C-NMR-Spektroskopie: Die ¹³C-NMR-Spektren wurden mit den Modellen *AV 300* (75 MHz) und *Fourier 300* (75 MHz) der Firma *Bruker* aufgenommen. Als interner Standard diente das Signal des Lösungsmittels (Dichlormethan-d₂: δ = 54.0 ppm, Toluol-d₈: δ = 137.9; 129.2; 128.3; 125.5; 20.4 ppm) wobei die chemischen Verschiebungen den ¹H-breitbandentkoppelten Spektren entnommen wurden.

⁷⁷Se-NMR-Spektroskopie: Die ⁷⁷Se-NMR-Spektren wurden mit dem *AV 300* (57 MHz) der Firma *Bruker* aufgenommen. Die Spektren wurden ¹H-breitbandentkoppelt vermessen. Die chemischen Verschiebungen sind in ppm angegeben.

Massenspektrometrie: El-Massespektren wurden am Gerät *Finnigan MAT 95-XP* der Firma *Thermo Electron* sowie ESI-TOF-Massespektren mit dem Modell *6210 Time-of-Flight LC*/*MS* der Firma *Agilent* aufgenommen.

### Autoklaven-Versuche der Rhodium-katalysierten Hydroformylierung

Die Hydroformylierung wurde in einem mit Druckkonstanthaltung, Gasflussmessung, Begasungsrührer und Druckpipette ausgestatteten 200 mL-Autoklaven der Fa. Premex Reactor AG, Lengau, Schweiz, durchgeführt. Das als Lösungsmittel verwendete Toluol wurde mit einem Pure Solv MD-7 System gereinigt und unter Argon aufbewahrt. Das als Substrat eingesetzte Substrat 1-Octen oder n-Octene (EVONIK Industries AG, Octenisomerengemisch aus 1-Octen: 3,3 %; cis+trans-2-Octen: 48.5%; cis+trans-3-Octen: 29.2%; cis+trans-Octen-4: 16.4%; gerüstisomere Octene: 2.6%) wurde mehrere Stunden über Natrium am Rückfluss erhitzt und unter Argon destilliert.

Für die Versuche wurden im Autoklaven unter Argonatmosphäre Lösungen der Katalysatorvorstufe und des Liganden gemischt. Als Katalysatorvorstufe kam [(acac)Rh(COD)] (*Umicore,* acac=Acetylacetonat-Anion; COD=1,5-Cyclooctadien) zum Einsatz. Für Versuche mit einer Konzentration von 100 ppm-m Rhodium wurden 10 mL einer 4.31 mM Lösung in den Autoklaven gegeben. Anschließend wurde die einem Verhältnis L/Rh = 5:1 (bzw. 1:1) entsprechende Masse des Liganden in 10 mL Toluol gelöst und zugemischt. Durch Zugabe von weiterem Toluol wurde das Anfangsvolumen der Katalysatorlösung auf 41.0 ml eingestellt. In eine druckfeste Pipette wurde eingefüllt: 1-Octen bzw. n-Octene (10.70 g). Der Autoklav wurde bei einem Gesamtgasdruck (Synthesegas: *Linde;* H₂(99.999%): CO (99.997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48.5 bar für einen Enddruck von 50 bar bzw. b) 19.5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. *Bronkhorst,* NL) über 4h geführt. Der Autoklav wurde nach Ablauf der Reaktionszeit auf Zimmertemperatur abgekühlt, unter Rühren entspannt und mit Argon gespült. Jeweils 1.0 mL der Reaktionsmischungen wurden unmittelbar nach Abschalten des Rührers entnommen, mit 5.0 mL Pentan verdünnt und gaschromatographisch analysiert: HP 5890 Series II plus, PONA, 50 m x 0.2 mm x 0.5 µm.

Einige Abkürzungen: Bn = Benzyl; ber. = berechnet; gef. = gefunden; MOM = Methylmethoxy;

### Synthese der Vorstufen:

### Allgemeine Arbeitsvorschrift - Diphenolselenide, Struktur (IX)

8.2 mmol des jeweiligen Phenols werden im entsprechenden Lösungsmittel gelöst (8.2 m). Das Reaktionsgemisch wird erhitzt und 4.9 mmol Selendioxid werden unter Rühren zugegeben. Das Lösungsmittel wird im Vakuum destilliert (Temperatur <70° C). Eine Fritte wird mit 2,5 cm Kieselgel (unten) und 2,5 cm Zeolith (oben) vorbereitet. Der Destillationsrückstand wird im Laufmittel aufgenommen und auf die Filtrationssäule geben. Mit Cyclohexan:Essigester (95:5) wird das Produkt von der Fritte waschen und in Fraktionen gesammelt. Die Fraktionen die das Produkt enthalten werden zusammengefasst und destillativ vom Laufmittel befreit. Die erhaltenen Fraktionen werden aus Cyclohexan:Essigester 95:5 umkristallisiert. Dazu wird der feste Rückstand bei 50 °C gelöst und unlösliche Rückstände über eine Glasfritte abfiltriert. Aus der gesättigten Lösung kristallisiert das Reaktionsprodukt bei Raumtemperatur über Nacht. Die erhaltenen Kristalle werden nochmal mit kaltem Cyclohexan gewaschen.

Die Strukturformel zeigt jeweils das bei der Reaktion angefallene Hauptprodukt.

### Bis(3,5-dimethyl-2-hydroxyphenyl)selen: Struktur IX, 1a

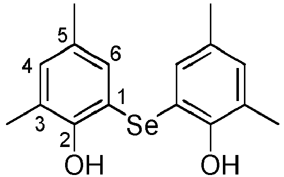

Die Durchführung der Reaktion erfolgt gemäß der allgemeinen Arbeitsvorschrift in einem verschraubbaren Reagenzglas. Hierzu werden 1.00 g (8.2 mmol, 1.0 Äquiv.) 2,4-Dimethylphenol und 0.54 g (4.9 mmol, 0.6 Äquiv.) Selendioxid in 1 mL Pyridin gelöst und erhitzt. Das Produkt wird als farbloser kristalliner Feststoff erhalten.

¹H-NMR (400 MHz, CDCl₃): δ (ppm) = 7.12 (s,2H, 6-H), 6.91 (s, 2H, 4-H), 5.97 (s,2H, OH), 2.23 (s, 6H, 3-CH₃) 2.23 (s, 6H, 5-CH₃); ¹³C-NMR (100 CDCl₃): δ (ppm) =151.7 (C-2),133.2 (C-3), 133.1 (C-5), 130.4 (C-4), 124.2 (C-6), 114.9 (C-1), 20.3 (5-CH₃), 16.5 (3-CH₃); ⁷⁷Se-NMR (76 MHz, CDCl₃): δ (ppm) = 163.36 ppm.

### Bis(3-tert-butyl-5-methyl-2-hydroxyphenyl)selen, Struktur IX, 1b

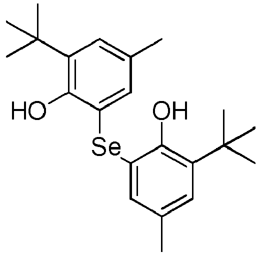

Die Durchführung der Reaktion erfolgt gemäß der allgemeinen Arbeitsvorschrift in einem verschraubbaren Reagenzglas. Dazu wurden 1.32 g (8.0 mmol, 1.0 Äquiv.) 2-*tert*-Butyl-4-methylphenol und 0.54 g (4.9 mmol, 0.6 Äquiv.) Selendioxid in 1 mL Pyridin gelöst und erhitzt.

¹H-NMR (300 MHz, CDCl₃): δ (ppm) = 7.15 (s, 2H, 6-H), 7.05 (s, 2H, 4-H), 5.07 (s,2H, OH), 2.21 (s, 6H, 5-CH₃), 2.21 (s, 18H, 3-C(CH₃)₃; ¹³C-NMR (75 MHz, CDCl₃): δ (ppm) = 152.1, 136.4, 133.4, 120.1, 129.5, 117.2, 35.1, 29.6, 20.8.

### 3,3',5,5'-Tetra-tert-butylbiphenyl-2,2'-diol, Struktur IX, 1c

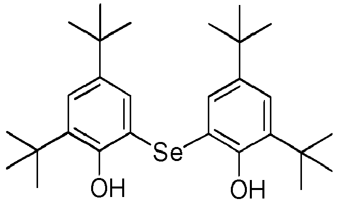

Die Durchführung der Reaktion erfolgt gemäß der allgemeinen Arbeitsvorschrift in einem verschraubbaren Reagenzglas. Dazu wurden 1.67 g (8.2 mmol, 1.0 Äquiv.) 2,4-Di-*tert*-butylphenol und 0.55 g (4.9 mmol, 0.6 Äquiv.) Selendioxid in 1 mL Pyridin gelöst und erhitzt.

¹H-NMR (400 MHz, CDCl₃): δ (ppm) = 7.31 (d, J=2.4 Hz, 2H), 7.29 (d, J=2.4), 6.29 (s, 2H), 1.42 (s, 18H), 1.24 (s, 18H); ¹³C-NMR (75 MHz, CDCl₃): δ (ppm) = 151.7, 143.5, 135.8, 129.8, 125.6, 117.2, 35.4, 34.4, 31.6, 29.7.

Biphenole: Die Synthese der Biphenole kann analog zu DE102013203865 und DE102013203867 erfolgen.

Synthese der Chlorophosphite: Die Synthese der Monochlorophosphite wie 6-Chlorodibenzo[d,f][1,3,2]dioxaphosphepin ist dem Fachmann bekannt und erfolgt nach bekannter Weise. Chlorophosphite lassen sich durch Zusatz von Phosphortrichlorit in Gegenwart einer Base aus den entsprechenden Dihydroxyverbindungen herstellen. Für nähere Informationen siehe auch "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### Synthese von 6-Chlorodibenzo[d,f][1,3,2]dioxaphosphepin IX, 2a

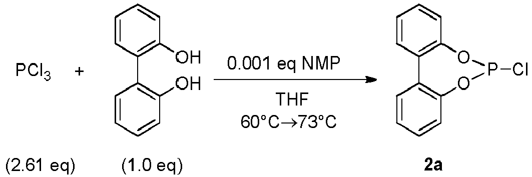

In einem ausgeheizten, unter Argon-Atmosphäre befindlichen 100 mL Dreihalskolben mit Rückflusskühler, Innenthermometer und Tropftrichter wurden 913 µL (1.43 g, 10.4 mmol, 2.61 eq) Phosphortrichlorid bei RT vorlegt und 3.80 µL *N*-Methyl-2-pyrrolidinon addiert. Das Reaktionsgemisch wurde auf 60°C Innentemperatur erhitzt. In einem separatem 10 mL Schlenkgefäß wurden 745 mg (4.00 mmol, 1.0 eq) 2,2-Biphenol in 20 mL abs. THF gelöst und innerhalb von 15 Minuten zur siedenden Reaktionslösung addiert. Es wurde mit 2.0 mL abs. THF nachgespült und auf eine Innentemperatur von 73°C erhitzt. Nach einer Reaktionszeit von 90 Minuten wurde die hellbraune Lösung auf RT abgekühlt und das Lösungsmittel unter verminderten Druck entfernt. Die braune Flüssigkeit wurde mit 10 mL abs. THF versetzt und zur Abtrennung des entstandenen Niederschlages filtriert. Das Lösungsmittel wurde erneut unter vermindertem Druck entfernt und das Rohprodukt drei Stunden im Vakuum bei 50°C getrocknet. Es wurden 962 mg (3.85 mmol, 96%, 99%ig) der Titelverbindung **2a** als dunkelbraune Flüssigkeit erhalten.

IR(ATR): *v̂* (cm⁻¹) = 3064; 3027; 2924; 2848; 2435; 1919; 1601; 1565; 1498; 1474; 1432; 1295; 1273; 1244; 1194; 1172; 1116; 1094; 1042; 1010; 942; 904; 855; 760; 738; 708; 614; 597; 579; 530; 514; 486; 470; 451; 428; ¹H-NMR (300 MHz, Toluol-d8): δ (ppm) = 7.29-6.83 (m, 8H, Ar-CH); ¹³C-NMR (75 MHz, Toluol-d₈): δ (ppm) = 149.3 (d, *J* = 5.7 Hz); 137.1; 130.9 (d, *J* = 3.5 Hz); 130.0 (d, *J* = 1.6 Hz); 129.3; 126.0 (d, *J* = 1.2 Hz); 122.0 (d, *J* = 2.2 Hz); ³¹P-NMR (122 MHz, Toluol-d₈): δ (ppm) = 180.5; C₁₂H₈ClO₂P (250.00 g/mol).

Entsprechend obiger Versuchsvorschrift können die folgende Monochlorophosphite der Struktur **IX** gemäss Tabelle 1 hergestellt werden.

**Tabelle 1: Monochlorophosphite IX**

| | | |
|---|---|---|
| 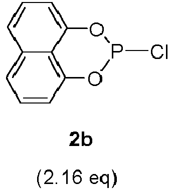 | 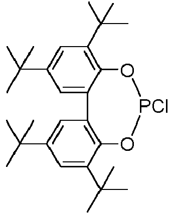 | 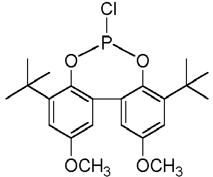 |
| Chloronaphtho[1,8-de][1,3,2]dioxaphosphinin | 2,2'-Bis-(3,5-ditert.-butyl)-phenol-chlorophosphit | 4,8-Di-tert-butyl-6-chloro-2,10-dimethoxydibenzo[d,f][1,3,2]dio xaphosphepin |
| 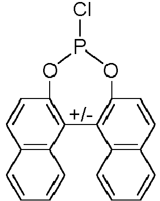 | 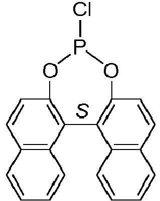 | 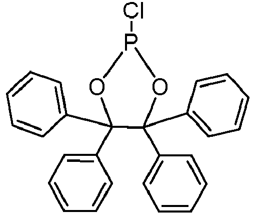 |
| rac-4-Chlor-dinaphtho[2,1-d:1',2'-f][1,3,2]dioxaphosphepin | 4-Chlor-S-dinaphtho[2,1-d:1',2'-f][1,3,2]dioxaphosphepin | 2-Chlor-4,4,5,5-tetraphenyl-1,3,2-dioxaphospholan |

### Die Synthese der Selen-Bisphosphite

### Synthese von Bis(2-(dibenzo[d,f][1,3,2]dioxaphosphepin-6-yloxy)-3,5-dimethylphenyl) selan, I (3a) und 2-((2-(Dibenzo[d,f][1,3,2]dioxaphosphepin-6-yloxy)-3,5-dimethylphenyl)-selanyl)-4,6-dimethylphenol, I (3b)

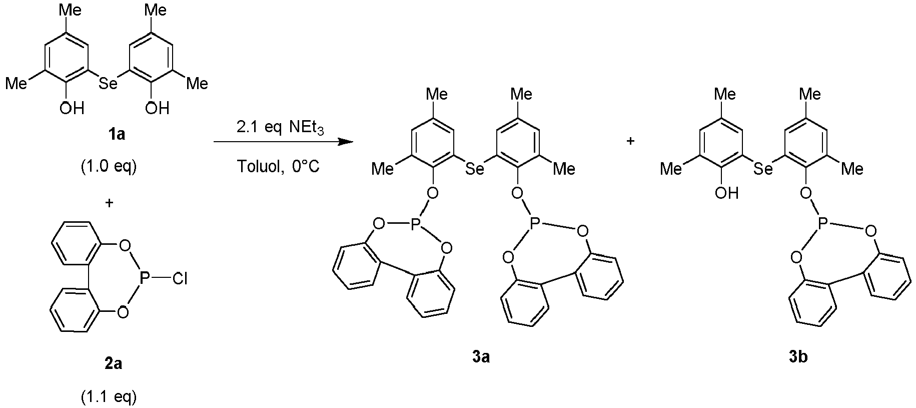

Es wurden 500 mg (1.56 mmol, 1.0 eq) Selenodiphenol **1a** und 460 µL (335 mg, 3.32 mmol, 2.13 eq) Triethylamin in 10 mL abs. Toluol gelöst und auf 0°C gekühlt. Anschließend wurden 858 mg (3.42 mmol, 2.2 eq, 90%ig)
6-Chlorodibenzo-[d,f][1,3,2]dioxaphosphepin **2a** zugetropft, wobei ein gallertartiger Niederschlag entstand. Die Reaktionslösung wurde über Nacht bei Raumtemperatur gerührt, über Kieselgel filtriert und das Lösungsmittel unter vermindertem Druck entfernt. Der erhaltene Feststoff wurde drei Stunden im Vakuum bei 50°C getrocknet. Es wurden 1.17 g (1.56 mmol, 100%) eines farblosen Feststoffes als Rohprodukt erhalten. 150 mg des Rohproduktes wurden säulenchromatographisch (2:1 H/Tol) gereinigt und ergaben 83.3 mg des Substanzgemisches aus den Titelverbindungen **3a** (88%ig im ³¹P-NMR) und **3b**.

HR-MS (ESI-TOF): ber. für C₄₀H₃₃O₆P₂Se ([M+H]⁺): 751.09160, gef.: 751.09143; ber. für C₄₀H₃₃O₆P₂SeNa ([M+Na]⁺): 773.07355, gef.: 773.07337.

### Synthese von Bis(3,5-dimethyl-2-(naphtho[1,8-de][1,3,2]dioxaphosphinin-2-yloxy)phenyl) selan, I 3c

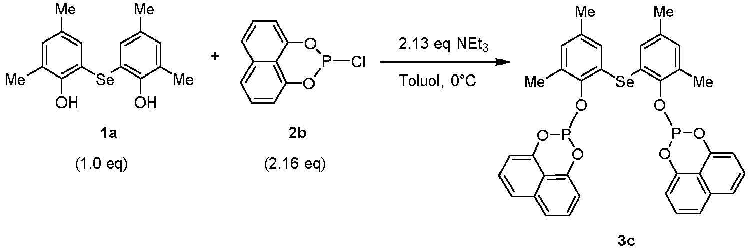

Es wurden 532 mg (1.66 mmol, 1.0 eq) Selenodiphenol **1a** und 490 µL (357 mg, 3.530 mmol, 2.13 eq) Triethylamin in 10 mL abs. Toluol gelöst und auf 0°C gekühlt. Anschließend wurden 804 mg (3.58 mmol, 2.16 eq, 92%ig) 2-Chloronaphtho-[1,8-de][1,3,2]dioxaphosphinin **2b** zugetropft, wobei ein gallertartiger Niederschlag entstand. Es wurde mit 1.0 mL abs. Toluol nachgespült und über Nacht bei Raumtemperatur gerührt. Das

Reaktionsgemisch wurde zur vollständigen Abtrennung des entstandenen Niederschlages filtriert, der Feststoff mit 20 mL abs. Toluol gewaschen und das Lösungsmittel unter vermindertem Druck entfernt. Das farblose Öl wurde drei Stunden im Vakuum bei 50°C getrocknet, in 45 mL n-Heptan aufgenommen und über Nacht bei RT gerührt. Der entstandene, farblose Niederschlag wurde abfiltriert und drei Stunden im Vakuum bei 50°C getrocknet. Es wurden 939 mg (1.35 mmol, 81 %, 99%ig) der Titelverbindung **3c** als farbloser Feststoff erhalten.

¹H-NMR (300 MHz, Dichlormethan-d₂): δ (ppm) = 7.58 (dd, *J* = 8.5 Hz, *J* = 1.0 Hz, 4H, Ar-CH); 7.41 (dd, *J* = 8.4 Hz, *J* = 7.5 Hz, 4H, Ar-CH); 7.05-6.90 (m, 6H, Ar-CH); 6.88-6.79 (m, 2H, Ar-CH); 2.21 (d, *J* = 0.8 Hz, 6H, -CH₃); 2.12 (d, *J* = 1.2 Hz, 6H, -CH₃); ¹³C-NMR (75 MHz, Dichlormethan-d₂): δ (ppm) = 148.4-147.1 (m), 144.4 (d, *J* = 3.0 Hz); 135.5; 135.3; 132.6; 132.1; 130.9; 127.6; 124.1-122.9 (m); 122.5; 117.1 (d, *J* = 14.1 Hz); 112.6; 20.64; 17.47; ³¹P-NMR (122 MHz, Dichlormethan-d₂): δ (ppm) = 107.6 Hz (*J*_{P-Se} = 46.8 Hz); HR-MS (ESI-TOF): ber. für C₃₆H₂₉O₆P₂Se ([M+H]⁺): 699.06023, gef.: 699.06008; ber. für C₃₆H₂₈O₆P₂SeNa ([M+Na]⁺): 721.04218, gef.: 721.04201.

### Hydroformylierung

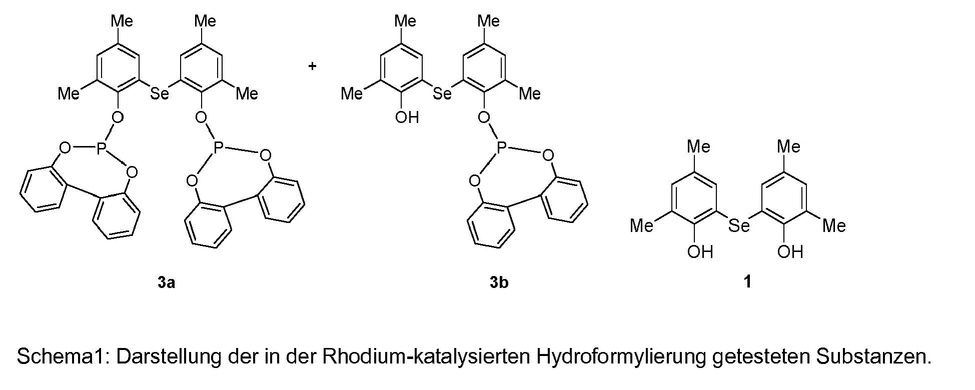

**Tabelle 2: Darstellung der Katalyse-Experimente unter der Verwendung von Organoselenverbindungen.**

| Eintrag | Ligand | Olefin /LM | Verhältnis Rh/ Ligand/Olefin | p [bar] | T [°C] | t[h] | S[%] |
|---|---|---|---|---|---|---|---|
| 1 | **3a/3b*** | 1-Octen / Toluol | 1:2:5461 (40 ppm Rh) | 50 | 100 | 4 | 80.3 |
| 2 | 1 | *n*-Octen / Toluol | 1:1:2197 (100 ppm Rh) | 50 | 120 | 4 | 33.2 |

| | | | | | | | |
|---|---|---|---|---|---|---|---|
| Erläuterungen zu Tabelle 2: p = Druck, T = Temperatur, t = Zeit, A = Ausbeute; S = n-Regioselektivität.*erfindungsgemäss | | | | | | | |

Durch Verwendung der erfindungsgemäßen Selena-Phosphite **3a/3b** kann eine Ausbeute mit hoher n-Regioselektivität von 80% erhalten werden erzielt werden.

Dem gegenüber zeigt die Rhodium-katalysierte Hydroformylierung mit Selenodiphenol **1** mit einer schlechten n-Regioselektivität von 33.2%.

Durch die Verwendung der erfindungsgemäßen Selena-Phosphite **3a/3b** konnte somit die gestellte Aufgabe erfüllt werden.

## Patentansprüche

1. Verbindung eines heterozyklischen Selena-Phosphites, welches eine allgemeine Struktur (**I**) aufweist 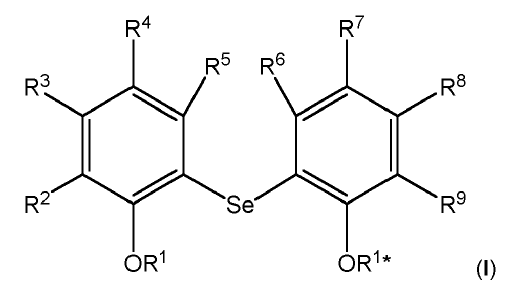 wobei R², R³, R⁴, R⁵, R⁶, R⁷, R⁸ und R⁹ jeweils unabhängig voneinander ausgewählt sind aus:-H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei die Gruppen -R¹ und -R^{1*} unabhängig ausgewählt sind aus -H und mindestens einer organofunktionellen Phosphit-Gruppe, wobei mindestens eine Gruppe von -R¹ und -R^{1*} einer organofunktionellen Phosphit-Gruppe entspricht.

2. Verbindung nach Anspruch 1, **dadurch gekennzeichnet, dass** im heterozyklischen Selena-Phosphit der allgemeinen Struktur (**I**)
a) mindestens -R¹ oder -R^{1*} einer organofunktionellen Phosphit-Gruppe entspricht und unabhängig ausgewählt ist aus den Strukturen (**II**), (**III**), (**IV**), (**V**), (**VI**) und (**VII**), 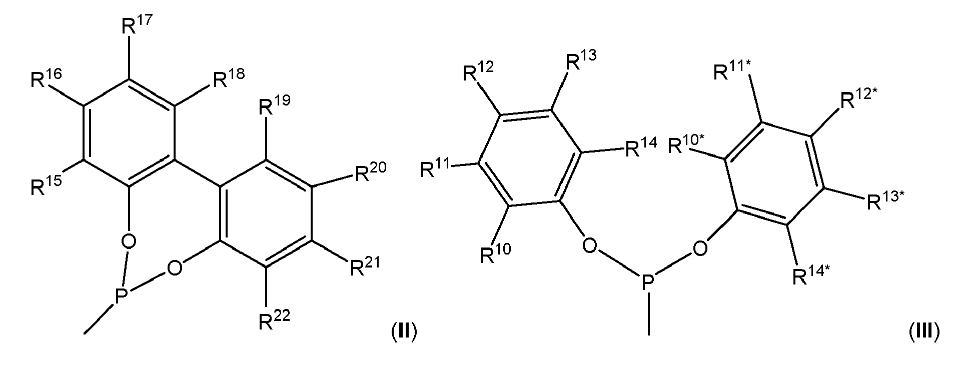 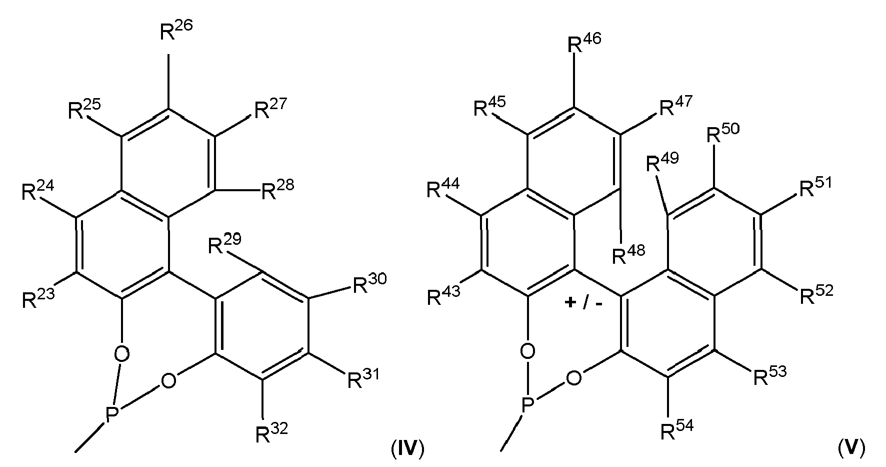 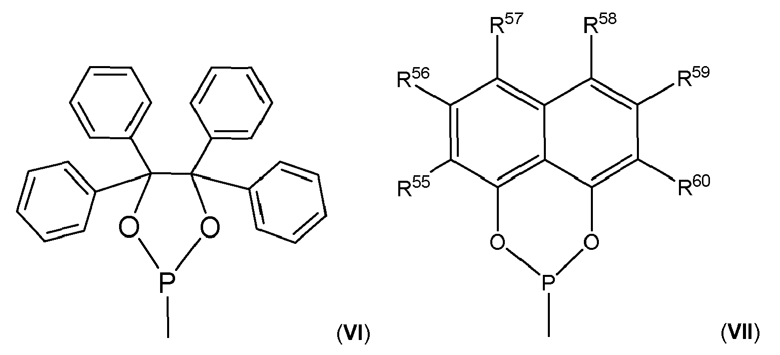 wobei die Reste
R¹⁵ R¹⁶, R¹⁷, R¹⁸, R¹⁹, R²⁰, R²¹, R²² in Struktur (**II**),
R¹⁰, R¹¹, R¹², R¹³, R¹⁴, R^{10*}, R^{11*}, R^{12*}, R^{13*}, R^{14*} in der Struktur (**III**),
R²³, R²⁴, R²⁵, R²⁶, R²⁷, R²⁸, R²⁹, R³⁰, R³¹ und R³² in Struktur (**IV**),
R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur (**V**),
R⁵⁵, R⁵⁶, R⁵⁷, R⁵⁸, R⁵⁹ und R⁶⁰ in Struktur (**VII**),
in der jeweiligen Struktur jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, und/oder
b) -R¹ und -R^{1*} gleich sind und einer organofunktionellen Phosphit-Gruppe entsprechen und ausgewählt sind aus den Strukturen (**II**), (**III**), (**IV**), (**V**), (**VI**) und (**VII**) oder
c) das Selena-Phosphit der Struktur (**I**) als Gemisch von a) und b) vorliegt.

3. Verbindung nach Anspruch 1 oder 2, **dadurch gekennzeichnet, dass** das heterozyklische Selena-Phosphit der allgemeinen Struktur (**I**) ausgewählt ist aus mindestens einer Verbindung der Struktur (**Ia**) 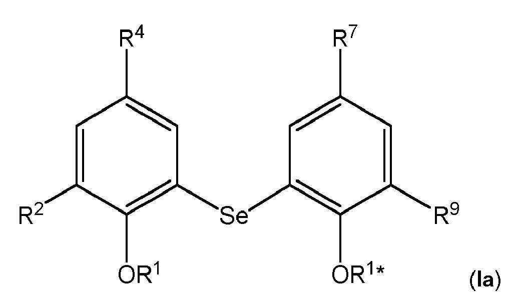 wobei R², R⁴, R⁷ und R⁹ jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen,
wobei -R¹ und -R^{1*} in Struktur (**Ia**) unabhängig ausgewählt sind aus -H und mindestens einer organofunktionellen Phosphit-Gruppe, wobei mindestens -R¹ oder -R^{1*} einer organofunktionellen Phosphit-Gruppe entspricht.

4. Verbindung nach einem der Ansprüche 1 bis 3, **dadurch gekennzeichnet, dass** das -R¹ und -R^{1*} im heterozyklischen Selena-Phosphit der allgemeinen Struktur (**I**) oder (**Ia**) ausgewählt sind aus den Strukturen **(II), (III), (IV),** (**V**), **(VI)** und (**VII**) 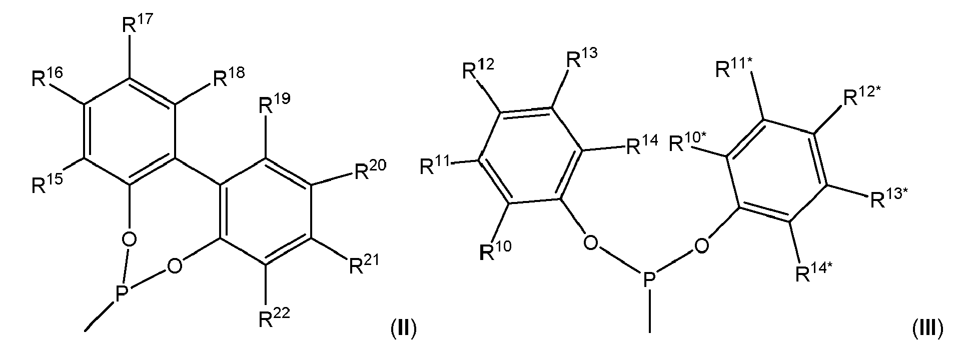 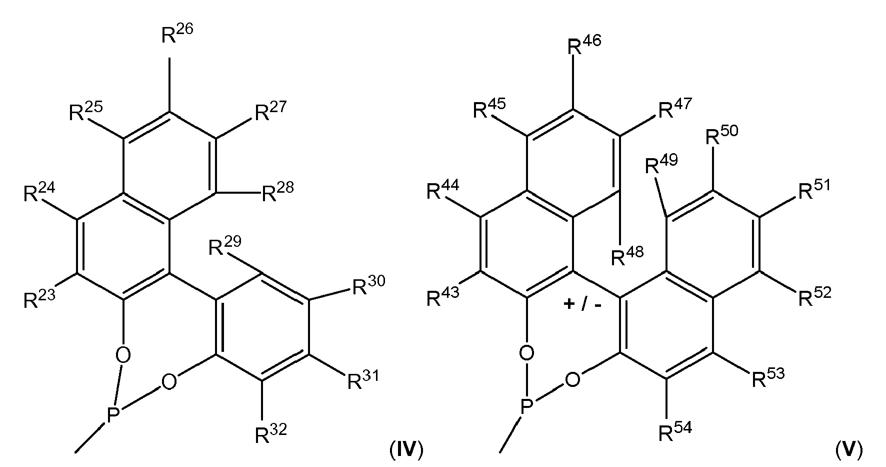 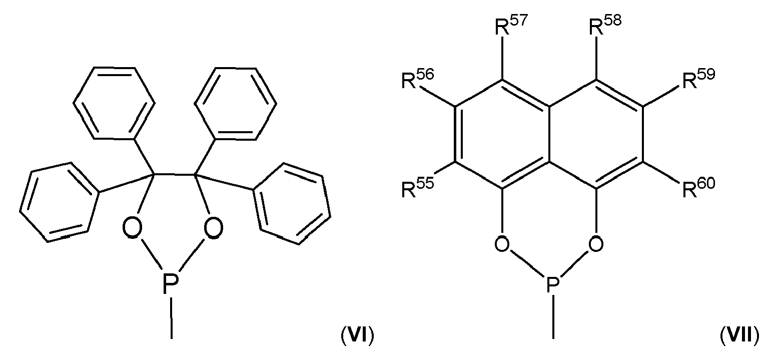 wobei die Reste
R¹⁵ R¹⁶, R¹⁷, R¹⁸, R¹⁹, R²⁰, R²¹, R²² -in Struktur (**II**),
R¹⁰, R¹¹, R¹², R¹³, R¹⁴, R^{10*}, R^{11*}, R^{12*}, R^{13*}, R^{14*} in der Struktur (**III**),
R²³, R²⁴, R²⁵, R²⁶, R²⁷, R²⁸, R²⁹, R³⁰, R³¹ und R³² in Struktur (**IV**),
R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur (**V**),
R⁵⁵, R⁵⁶, R⁵⁷, R⁵⁸, R⁵⁹ und R⁶⁰ in Struktur (**VII**),
in der jeweiligen Struktur jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen.

5. Verbindung nach einem der Ansprüche 1 bis 4, **dadurch gekennzeichnet, dass** -R¹ und -R^{1*} im heterozyklischen Selena-Phosphit der allgemeinen Struktur (**I**) oder (**Ia**) ausgewählt sind aus der Struktur (**II**) 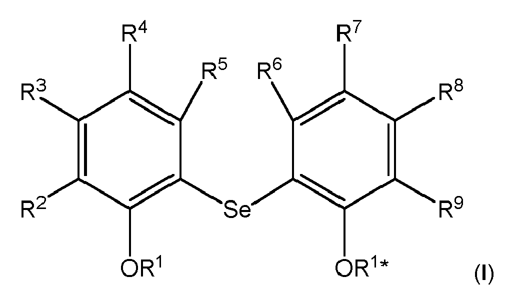 wobei R², R³, R⁴, R⁵, R⁶, R⁷, R⁸ und R⁹ jeweils unabhängig voneinander ausgewählt sind aus:-H, -(C₁-C₁₂)-Alkyl, -O-(C₁-C₁₂)-Alkyl, oder 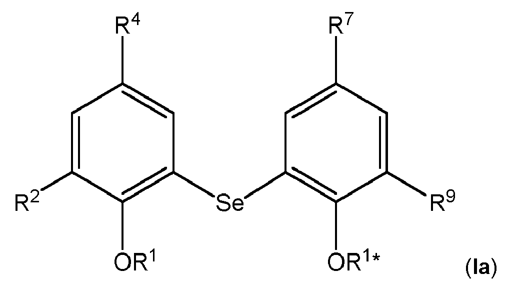 wobei R², R⁴, R⁷ und R⁹ jeweils unabhängig voneinander ausgewählt sind aus: -(C₁-C₁₂)-Alkyl, -O-(C₁-C₁₂)-Alkyl, und 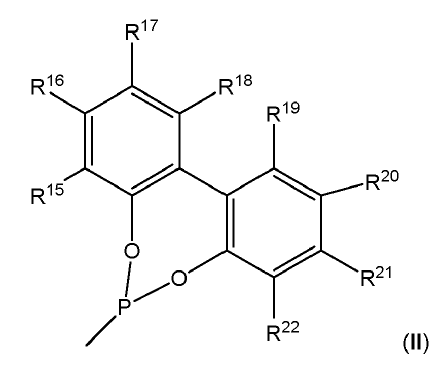 mit R¹⁵, R¹⁶, R¹⁷, R¹⁸, R¹⁹, R²⁰, R²¹, R²² in Struktur **(II),** jeweils unabhängig voneinander ausgewählt sind aus: -H, -(C₁-C₁₂)-Alkyl, -O-(C₁-C₁₂)-Alkyl,
wobei -R¹ und -R^{1*} in Struktur (**I**) oder (**Ia**) unabhängig ausgewählt sind aus -H und mindestens einer organofunktionellen Phosphit-Gruppe, wobei mindestens eine Gruppe von -R¹ und -R^{1*} einer organofunktionellen Phosphit-Gruppe entspricht.

6. Verbindung nach einem der Ansprüche 1 bis 5, **dadurch gekennzeichnet, dass** das -R¹ und -R^{1*} ausgewählt sind aus den Alternativen
a) -R¹ und -R^{1*} sind in Struktur (**I**) oder (**Ia**) gleich und ausgewählt aus (**II**), (**III**), (**IV**), (**V**), (**VI**) und (**VII**),
b) in Struktur (**I**) oder (**Ia**) ist -R¹ gleich -H und -R^{1*} ist ausgewählt aus (**II**), (**III**), (**IV**), (**V**), (**VI**) und (**VII**),
c) in Struktur (**I**) oder (**Ia**) ist -R^{1*} gleich -H und -R¹ ist ausgewählt aus (**II**), (**III**), (**IV**), (**V**), (**VI**) und (**VII**),
d) die Verbindung der Alternative a) liegt im Gemisch mit b) und/oder c) vor.

7. Verfahren zur Herstellung mindestens eines heterozyklischen Selena-Phosphites der allgemeinen Struktur (**I**) 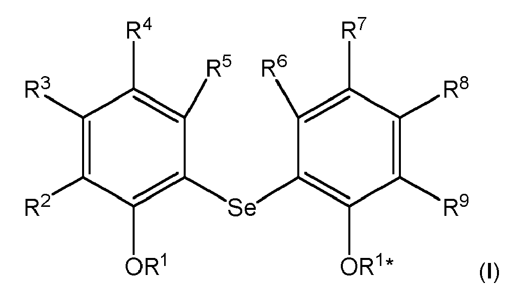 wobei R², R³, R⁴, R⁵, R⁶, R⁷, R⁸ und R⁹ jeweils unabhängig voneinander ausgewählt sind aus: - H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei die Gruppen -R¹ und -R^{1*} in Struktur (**I**) unabhängig ausgewählt sind aus -H und mindestens einer organofunktionellen Phosphit-Gruppe, wobei mindestens eine Gruppe von-R¹ und -R^{1*} einer organofunktionellen Phosphit-Gruppe entspricht,
umfassend mindestens den Verfahrensschritt
(i) Umsetzen eines Selenodiaryls der allgemeinen Struktur (**IX**) 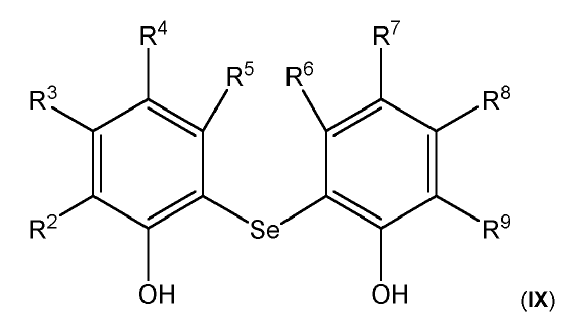 wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
(ii) mit mindestens einer Halogenphosphit-Verbindung R¹Hal der Formel (**Xa**) und/oder R¹*Hal der Formel (**Xb**), wobei Hal jeweils unabhängig ausgewählt ist aus Fluor, Chlor, Brom, Jod, wobei -R¹ und -R^{1*} in Formel **Xa** und/oder **Xb** jeweils unabhängig einer organofunktionellen Phosphit-Gruppe entsprechen,
(iii)und erhalten mindestens eines heterozyklischen Selena-Phosphits der allgemeinen Struktur (I) oder eines Gemisches von Selena-Phosphiten der Struktur (**I**).

8. Verfahren nach Anspruch 7, **dadurch gekennzeichnet, dass** in den Halogenphosphit-Verbindungen R¹Hal der Formel (**Xa**) und/oder R¹*Hal der Formel (**Xb**) Hal jeweils unabhängig ausgewählt ist aus Fluor, Chlor, Brom, Jod, und
a) -R¹ oder -R^{1*}jeweils unabhängig ausgewählt sind aus den Strukturen (**II**), (**III**), (**IV**), (**V**), (**VI**) und (**VII**) 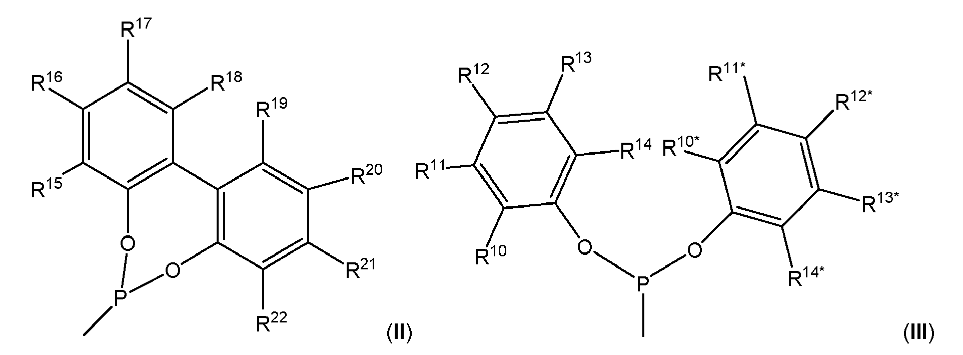 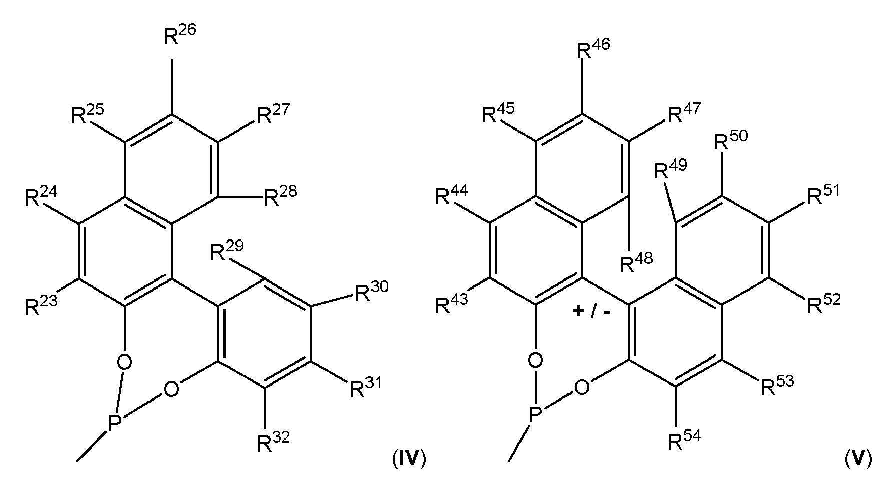 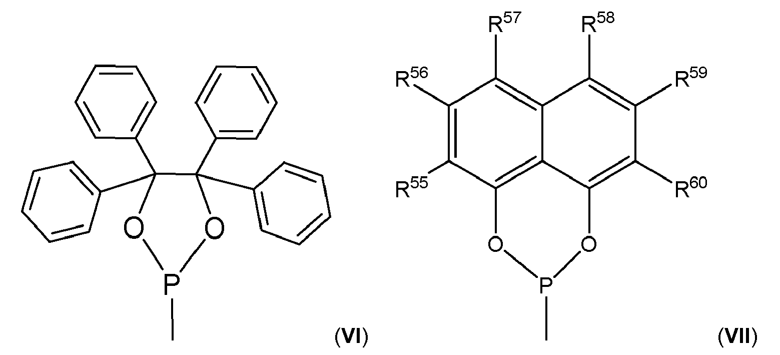 wobei die Reste
R¹⁵ R¹⁶ R¹⁷, R¹⁸, R¹⁹, R²⁰, R²¹, R²² -in Struktur (**II**),
R¹⁰, R¹¹, R¹², R¹³, R¹⁴, R^{10*}, R^{11*}, R^{12*}, R^{13*}, R^{14*} in der Struktur (**III**),
R²³, R²⁴, R²⁵, R²⁶, R²⁷, R²⁸, R²⁹, R³⁰, R³¹ und R³² in Struktur (**IV**),
R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur (**V**),
R⁵⁵, R⁵⁶, R⁵⁷, R⁵⁸, R⁵⁹ und R⁶⁰ in Struktur (**VII**),
jeweils in den Strukturen unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, oder
b) -R¹ und -R^{1*} gleich sind und ausgewählt sind aus den Strukturen (**II**), (**III**), (**IV**), (**V**), (**VI**) und (**VII**)**.**

9. Verfahren nach Anspruch 7 oder 8, **dadurch gekennzeichnet, dass** die Halogenphosphit-Verbindungen R¹Hal der Formel (**Xa**) und R^{1*}Hal der Formel (**Xb**) gleich sind, und
Hal ausgewählt ist aus Chlor oder Brom, und
-R¹ und -R^{1*} ausgewählt sind aus den Strukturen (**II**), (**III**), (**IV**), (**V**), (**VI**) und (**VII**) 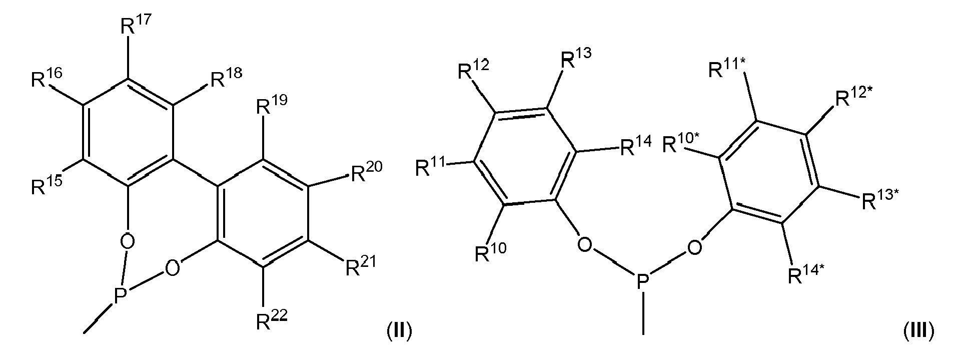 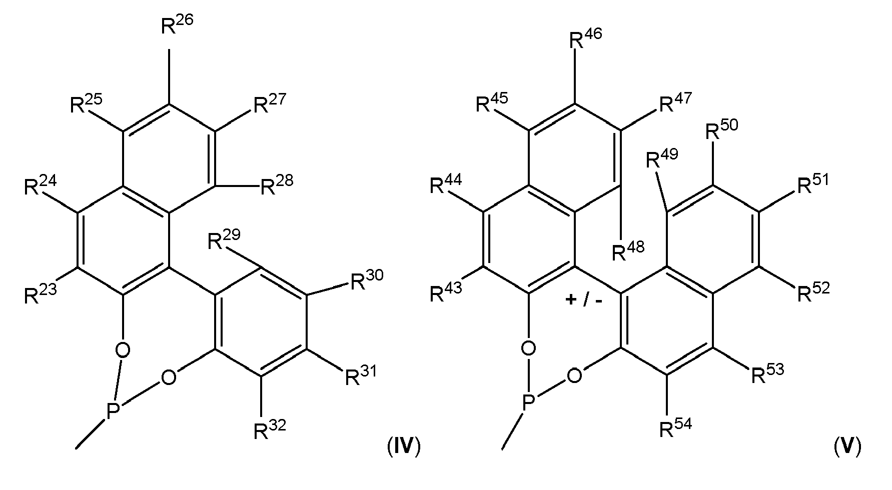 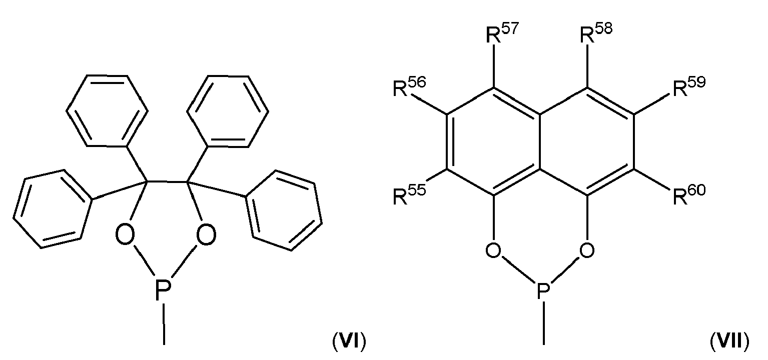 wobei die Reste
R¹⁵ R¹⁶, R¹⁷, R¹⁸, R¹⁹, R²⁰, R²¹, R²² -in Struktur (**II**),
R¹⁰, R¹¹, R¹², R¹³, R¹⁴, R^{10*}, R^{11*}, R^{12*}, R^{13*}, R^{14*} in der Struktur (**III**),
R²³, R²⁴, R²⁵, R²⁶, R²⁷, R²⁸, R²⁹, R³⁰, R³¹ und R³² in Struktur (**IV**),
R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur (**V**),
R⁵⁵, R⁵⁶, R⁵⁷, R⁵⁸, R⁵⁹ und R⁶⁰ in Struktur (**VII**),
in der jeweiligen Struktur jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen.

10. Verfahren nach einem der Ansprüche 7 bis 9, **dadurch gekennzeichnet, dass** das Selenodiaryl der allgemeinen Struktur (**IXa**) entspricht 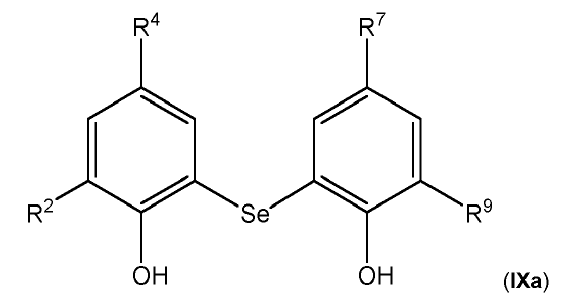 wobei R², R⁴, R⁷ und R⁹ jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen.

11. Verfahren nach einem der Ansprüche 7 bis 10, wobei (i) die Umsetzung in Gegenwart einer Base, insbesondere eines Amins oder einer Pyridinbase.

12. Komplex umfassend
- mindestens eine Verbindung der allgemeinen Struktur (**I**) oder (**Ia**) der Ansprüche 1 bis 6 und
- mindestens ein Metallatom ausgewählt aus Rh, Ru, Co, Ir.

13. Verwendung einer Verbindung nach einem der Ansprüche 1 bis 6 als Ligand in einem Komplex umfassend mindestens ein Metallatom.

14. Verwendung einer Verbindung nach einem der Ansprüche 1 bis 6 zur Katalyse einer Hydroformylierungsreaktion oder als Zwischenprodukt zur Herstellung von Liganden.

15. Verfahren umfassend die Verfahrensschritte
(i) Vorlegen mindestens eines Olefins,
(ii) Zugabe eines Komplexes nach Anspruch 12, oder einer Verbindung nach einem der Ansprüche 1 bis 6 und einer Substanz, welche ein Metallatom ausgewählt aus Rh, Ru, Co, Ir aufweist,
(iv) Zuführen von H₂ und CO,
(v) Erwärmen des Reaktionsgemisches, wobei das Olefin zu einem Aldehyd umgesetzt wird.
